# EUROPEAN PATENT APPLICATION

(11) **EP 0 982 036 A1**
(43) Date of publication of application: **01.03.2000**
(21) Application number: 98116284.5
(22) Date of filing: 28.08.1998
(51) Int. Cl.: A61K 38/17, A61K 38/49, A61K 39/395

(54) **Modulation of Beta2 -integrin-mediated cell adhesion**

(71) Applicant: Wilex Biotechnology GmbH, 81675 München (DE)
(72) Inventor: The designation of the inventor has not yet been filed
(74) Representative: Weiss, Wolfgang, Dipl.-Chem. Dr.

(57) **Abstract**

According to the present invention it was found that the urokinase receptor induces the β₂-integrin-mediated activation of leukocytes and tumor cells, leading to an adhesion of these cells to endothelial cells, a pathway which plays an important role in hyperinflammatory conditions and the formation of metastases. Thus, the urokinase receptor and ligands thereof, e.g. antibodies, peptides or lower molecular compounds, are suitable as medicaments for the treatment of these diseases. By using different uPAR ligands leukocyte adhesion can either be stimulated or inhibited.

## Description

The present invention refers to the use of urokinase receptors and ligands thereof for the modulation of β₂-integrin-mediated cell-to-cell adhesion. Further, new medicaments for the treatment of inflammatory conditions and tumors are provided.

Leukocyte activation and adhesion to the endothelium are pivotal steps for their recruitment to inflamed tissue sites in order to initiate an adequate inflammatory response. Leukocyte adhesion and transmigration are highly coordinated multistep processes^{1,2} that require tight regulation by e.g. the expression and upregulation of various adhesion receptors on vascular and circulatory blood cells. Due to uncontrolled leukocyte activation hyperinflammatory pathological states are associated with the development of vascular diseases such as atherosclerosis and restenosis. In particular, the β₂-integrin family that consists of the four known members LFA-1 (αLβ₂, CD11a/CD18), Mac-1 (αMβ₂, CD11b/CD18, CR3), p150,95 (αXβ₂, CD11c/CD18), and αDβ₂ (CD11d/CD18) plays a major role in the adhesion of different leukocyte subtypes to the vessel wal1^{3,4}. LFA-1 and Mac-1 represent important mediators of leukocyte adhesion to endothelium in acute inflammation. While Mac-1 is constitutively expressed on neutrophils and monocytes, LFA-1 is predominantly expressed on lymphocytes, but recent data underline its important contribution in neutrophil recruitments^{5,6}. Following activation, Mac-1 and LFA-1 firmly bind to ICAM-1 (CD54) expressed on vascular endothelial¹ and smooth muscle cells⁷. Leukocyte activation via cytokines, chemoattractants, or phorbolester induces both conformational changes in β₂-integrins necessary for enhanced ligand recognition as well as translocation of Mac-1 to the cell surface⁸. Direct integrin activation can also be achieved extracellularly by divalent cations such as Mn²⁺⁹. β₂-integrins have been reported to form complexes with other plasma membrane proteins such as CD63¹⁰, the immunoglobulin receptor FcyRIIIB (CD16b)¹¹ and the urokinase receptor (uPAR, CD87)^{12,13} suggesting possible functional interaction. Yet, the mode of molecular interactions in this receptor cross-talk is unknown.

uPAR consists of three homologous domains and is anchored to the plasma membrane by a glycolipid moiety that is susceptible to dissociation by phosphatidylinositol-specific phospholipase C¹⁴. Intact uPAR binds the protease uPA (urokinase-type plasminogen activator) as well as the adhesive protein vitronectin with high affinity¹⁵ and thereby plays a critical role in pericellular proteolysis¹⁶ and modulation of cellular contacts^{17,18}. The expression level of uPAR on various cells strongly correlates with their migratory and invasive potential¹⁹. Although uPAR lacks an own transmembrane and cytoplasmic domain, uPA-binding has been reported to transduce signals to the cell interior in e.g. leukocytes resulting in calcium mobilization ²⁰, protein kinase phosphorylation ^{12,21-23}, or altering leukocyte behaviour with respect to chemotaxis-induction, superoxide anion production and synthesis of matrix metalloproteinases^{20,23,24}. For some of these functions uPAR is suggested to use related transmembrane integrins as signal transduction devices²⁵. Recently, it has been shown that uPAR can alter the function of β₁-integrins²⁶ and that its presence favours monocyte adhesion to and degradation of fibrinogen, one of the adhesive ligands of Mac-1^{27,28}. Preliminary in vitro results³⁹ indicate that soluble uPAR is able to modulate the monocytic cell adhesion to endothelial cells via modulation of the binding activity of Mac-1.

Despite these findings, which show an interaction between urokinase receptor and integrins, their function or the biochemical pathway following therefrom was entirely unclear. According to the present invention it was found that the urokinase receptor and ligands thereof are able to modulate the β₂-integrin-mediated activation of leukocytes and tumor cells in vivo. Thus, the urokinase receptor and particularly ligands thereof, e.g. antibodies, peptides or lower molecular compounds, are suitable as medicaments for the treatment of these diseases. By using different uPAR ligands leukocyte adhesion can either be stimulated or inhibited.

Thus, the subject matter of the present invention is the use of urokinase receptor and ligands thereof for the manufacture of a medicament to modulate the β₂-integrin-mediated cell-to-cell adhesion, particularly the adhesion of leukocytes such as lymphocyte-derived cells, e.g. activated T-cells or B-lymphocytes, granulocytes, e.g. neutrophils or eosinophils and/or monocytes, or tumor cells, e.g. metastating tumor cells. By the term "modulation" a stimulation and/or inhibition of cell adhesion is understood.

The present invention provides a means for modulating β₂-integrin-mediated cell-to-cell adhesion, i.e. adhesion of urokinase receptor and β₂-integrin carrying cells, which may be present as single cells in the blood stream, to target cells in vivo. The target cells are selected from cells to which a β₂-integrin-mediated adhesion can take place. More preferably, the target cells are endothelial cells, e.g. vascular endothelial cells, and muscle cells, e.g. smooth muscle cells. It should be noted that the present invention also provides means for modulating β₂-integrin- mediated adhesion of cells to other surfaces, e.g. to surfaces of body implants.

In one embodiment of the present invention the β₂-integrin-mediated cell adhesion is modulated by urokinase receptor itself. It should be understood that the term "urokinase receptor" in this context also comprises fragments thereof including peptide fragments.

In a further and preferred embodiment the β₂-integrin-mediated cell adhesion is modulated by urokinase receptor ligands. Ligands for the urokinase receptor are known. Examples thereof are anti-urokinase receptor antibodies, e.g. polyclonal or monoclonal antibodies, antibody fragments or single-chain antibodies. Examples of suitable antibodies are disclosed in WO 90/12091. Further examples for suitable urokinase receptor ligands are urokinase and urokinase receptor binding fragments thereof, particularly peptide fragments, examples of which are disclosed in Appella et al. (J. Biol. Chem. 262 (1987), 4437-4440), Magdolen et al. (Eur. J. Biochem. 237 (1996), 743-751), Goretzki et al. (Fibrinolysis and Proteolysis 11 (1997), 11-19) and WO 94/22464. Further examples are synthetic peptides or lower molecular chemical compounds capable of binding urokinase receptor, examples of which are disclosed in Goodson et al. (Proc. Natl. Acad. Sci. USA 91 (1994), 7129-7133).

Especially preferred are cyclic peptides according to PCT/EP98/02179. These peptides may have the general formula (I): 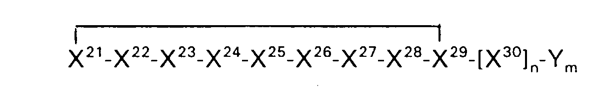 wherein
X²¹-X³⁰ each represent an amino carboxylic acid and X²¹ and X²⁹ are linked with each other,
Y is a spacer,
n and m are each independently 0 or 1,
and the monomeric building blocks are linked via -NR¹CO- or -CONR¹-bonds, wherein R¹ in each case independently represents hydrogen, methyl or ethyl, and pharmaceutically acceptable salts and derivatives thereof.

More preferably, amino acid residues X²¹-X³⁰ in each case independently have the following meanings:
(i) X²¹ and X²⁹ are residues having an SH side chain or residues linked via a thioether bond, e.g. Cys;
(ii) X²² and X²⁷ are residues having an aliphatic side chain, e.g. an aliphatic hydrophilic side chain, particularly preferred an amido side chain, e.g. Asn or GIn, especially Asn;
(iii) X²³ is a residue having a basic or aliphatic hydrophilic side chain, e.g. Lys, Orn, Arg, GIn or Asn, especially Lys;
(iv) X²⁴, X²⁵ and X³⁰ are residues having aromatic side chains, e.g. Tyr, Phe or Trp, especially X²⁴ = Tyr, X²⁵ = Phe and X³⁰ = Trp;
(v) X²⁶ is a residue having an aliphatic side chain, preferably an aliphatic hydrophilic side chain, e.g. hydroxyvaline, homoserine, Ser or Thr, especially Ser; and
(vi) X²⁸ is a residue having an aliphatic side chain, preferably an aliphatic hydrophobic side chain, e.g. Val, norvaline, norleucine, IIe, Leu or Ala, especially Ala.

Further, these peptides may be cyclic peptides having a nine-membered ring, wherein at least 2 and preferably at least 3 amino acids in the ring have a sequence derived from region of amino acids 22-28 from uPA.

Finally, these peptides may have the general formula (II):

X¹-[X²]ₙ-[X³]ₘ-X⁴-K-Y-F-X⁵-X⁶-I-X⁷-W-[X⁸]ᵣ

wherein X¹-X⁸ each represent an amino carboxylic acid,
n, m and r are each independently 0 or 1,
K, F, I and W, respectively, are α-amino carboxylic acids having Lys, Phe, IIe and Trp-side chains, respectively, and the monomeric building blocks are linked via -NR¹CO- or -CONR¹- bonds, wherein R¹ in each case independently represents hydrogen, methyl or ethyl, and pharmaceutically acceptable salts and derivatives thereof, wherein at least one of the residues X¹, X², X³, X⁶, I, X⁷ and X⁸ are a D-amino acid residue and X¹ and X⁷ or X¹ and X⁸ may be linked with each other.

The present invention describes the essential role of urokinase receptor and its ligands for β₂-integrin-dependent cell adhesion on the basis of the results that (i) dissociation of urokinase receptor from the cell surface abrogated β₂-integrin-dependent adhesion of leukocytes to endothelial cells in response to cell activation or direct integrin activation, (ii) readdition of soluble intact, but not truncated urokinase receptor could totally rescue β₂-integrin-mediated adhesion, and (iii) urokinase receptor occupation by a specific monoclonal antibody that is known to interfere with urokinase binding to domain I strongly induces leukocyte adhesion to vascular endothelial and smooth muscle cells, whereas urokinase itself inhibited β₂-integrin activation-dependent adhesion. Further in vivo data are provided which demonstrate that the uPAR-β₂-integrin interaction is subject to modulation also in living organisms.

Taken together, these findings suggest that the urokinase receptor/urokinase complex is essential to maintain the dynamics of the cell-to-cell and cell-to-matrix interactions at various stages of the leukocyte recruitment process. Consequently, disturbance of this balanced molecular network by e.g. changes of the expression level of uPAR or the local concentration of uPA may result in disease stages as observed in peritonitis, hyperinflammatory situations or atherogenesis.

Urokinase receptor is capable of facilitating and controlling integrin activation in response to β₂-integrin agonists, but also of inducing integrin activation on its own when stimulated by suitable anti-urokinase receptor antibodies. Antibody ligation may result in induction of adherence of monocytic cells as well as neutrophils to vascular endothelial and smooth muscle cells. Urokinase was found to interfere either by competing for antibody binding or by not allowing integrin activation to occur.

These findings also indicate that urokinase receptor plays a major role for leukocyte inflammatory reactions by facilitating and modulating adhesion to the endothelium provided the receptor is intact either in its cell-bound or soluble form.

Hence, by the present invention a medicament is provided which is suitable for the treatment of diseases associated with β₂-integrin-mediated cell adhesion. Particularly, the present invention provides means for the treatment of inflammatory conditions, immune diseases or vascular diseases, e.g. peritonitis, hyperinflammatory conditions such as septic shock or atherogenesis. Further, the present invention provides means for the treatment of invasive processes, e.g. tumors, particularly for the inhibition of tumor metastasis.

The administration mode and the administered dose of the respective medicament firstly depend on the medicament used and secondly on the type and severity of the disease to be treated. For example, the medicament may be administered in one or several doses orally and/or parenterally. The person skilled in the art can easily determine the respective suitable dose. For example, the daily dose may be in the rage of 1 µg to 100 mg per kg body weight. Apart from the active agent, the medicament may also comprise further conventional pharmaceutical carriers, fillers, diluents or adjuvants.

The present invention is further illustrated by the following figures and examples.

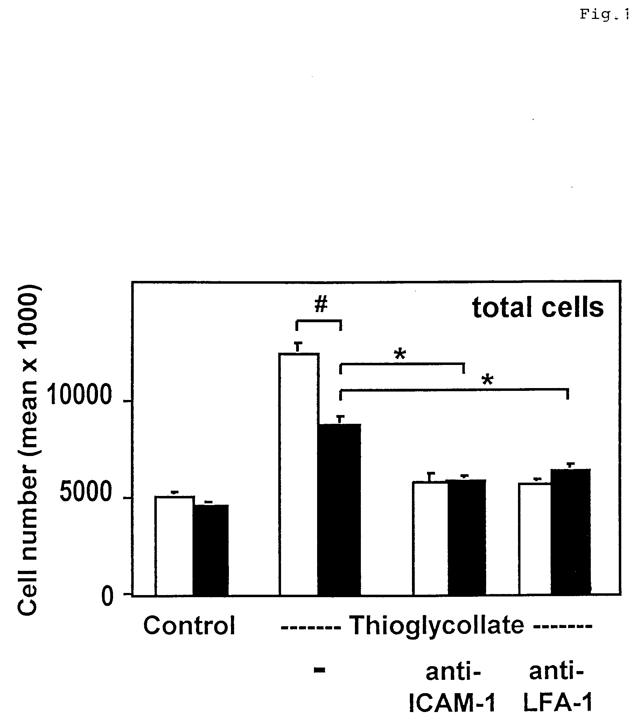

- Figure 1:: Figure 1: Leukocyte emigration in thyoglycollate-induced peritonitis. Wild-type mice (white bars) and uPAR-deficient mice (black bars) were injected intraperitoneally with buffer alone (control) or with thyoglycollate solution in the absence or presence of the indicated antibodies. After leukocyte emigration for 4 h, mice were killed and the leukocytes were counted in lavages from the peritoneum. SE was calculated from 4 animals for each condition and the experiment was repeated twice ^{#}P<0.02; *P<0.01

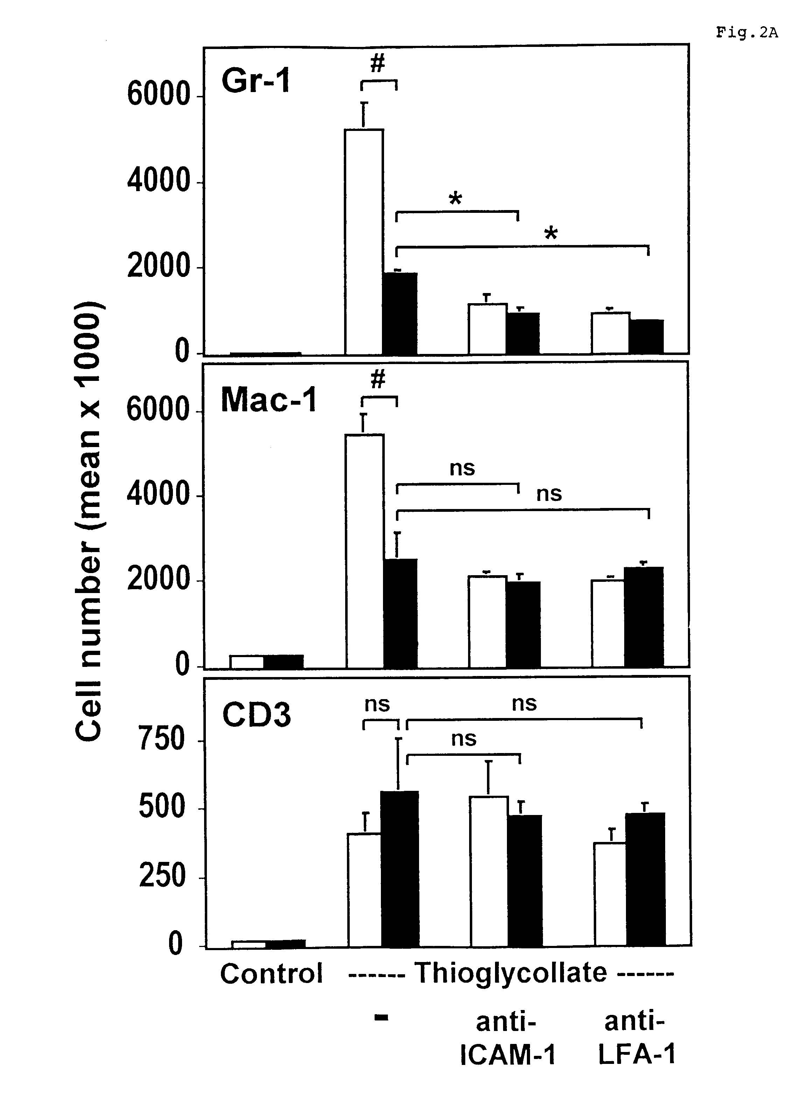

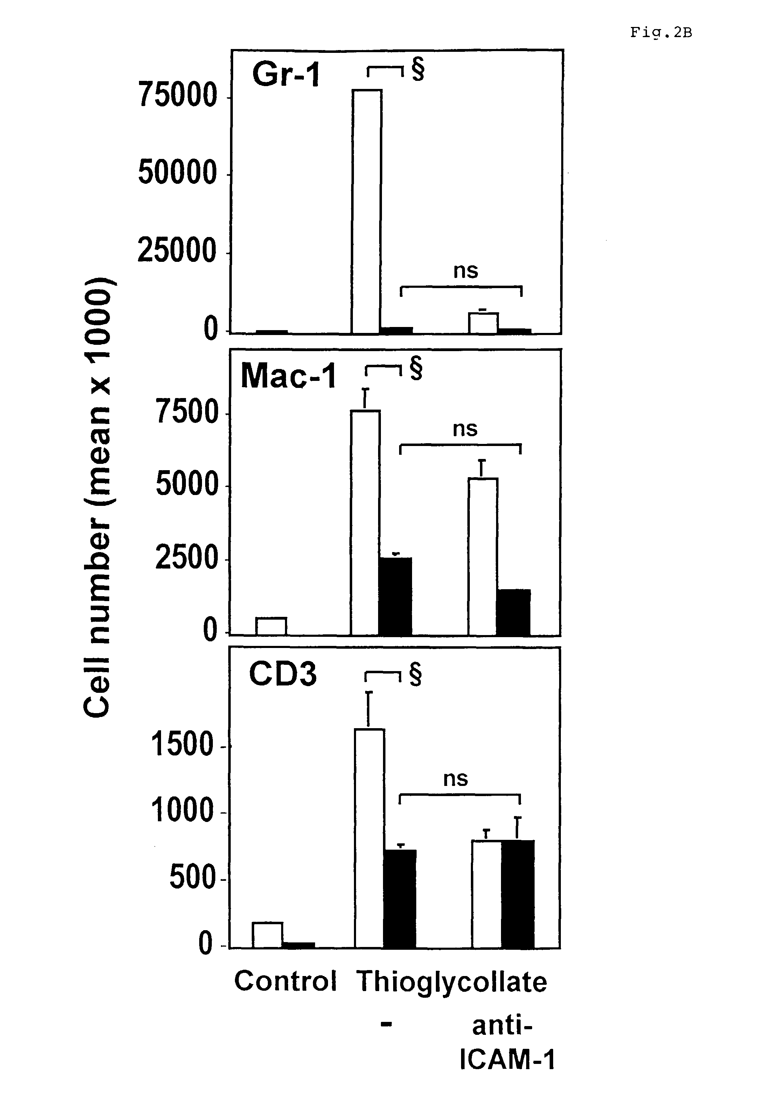

- Figure 2:: Analysis of subpopulations of emigrated leukocytes in the peritoneal lavage. Leukocytes obtained in peritoneal lavages after induction of peritonitis for 4 (A) or 24 h (B) from wild-type mice (white bars) and uPAR-deficient mice (black bars) were analyzed by flow cytometry for the expression of Gr-1 (anti-granulocytes), Mac-1 (anti-myeloid cells) or CD3 (anti-CD3 TCR complex). Absolute numbers of cells were calculated from the percentage of stained cells and the number of total emigrated cells shown in Fig.1.^{#}P<0.01; *P<0.002; ^{§}P<0.005.

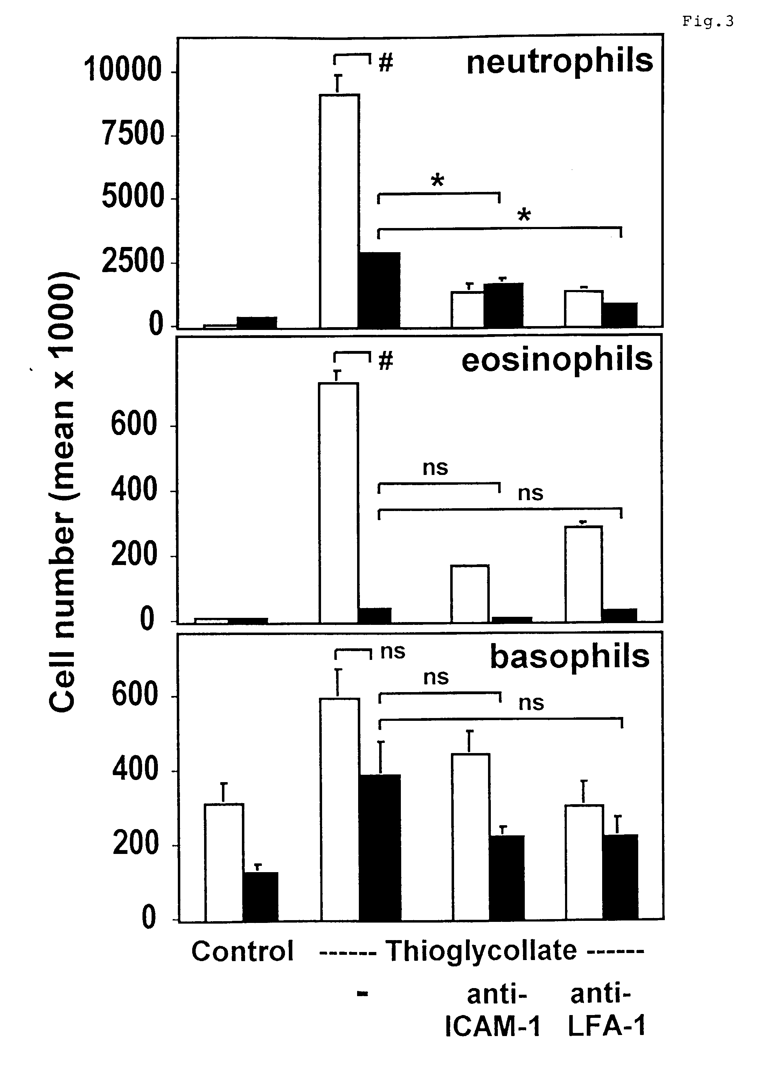

- Figure 3:: Quantitation of granulocyte subpopulations in the peritoneal lavage. The migrated leukocytes from wild-type mice (white bars) and uPAR-deficient mice (black bars) were cytocentrifuged and stained with May-Grünwald-Giemsa solution. The quantitation of cell numbers of leukocyte subpopulations was performed by light microscopy. SE was calculated from 4 animals for each condition and the experiment was repeated twice. ^{#}P<0.02; *P<0.005.

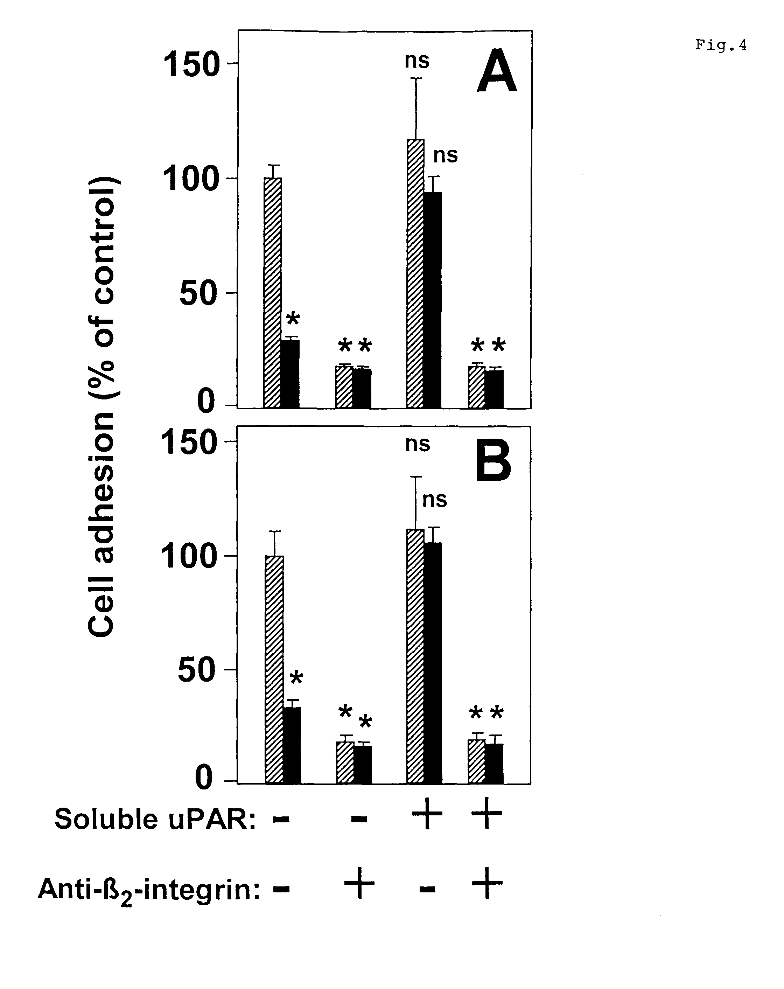

- Figure 4:: Requirement of uPAR for leukocyte adhesion to endothelial cells. Myelo-monocytic HL60-cells were either not treated (hatched bars) or pretreated with pi-PLC (0.5 U/mL, 90 min, 37°C) (black bars), washed, and incubated for 10 min with or without soluble intact uPAR (0.8 µg/mL), washed and stimulated for 20 min with (A) PMA (10 ng/mL) or (B) Mn²⁺ (1.0 mmol/L). After another washing step the adhesion assay was performed in the presence or absence of anti-β₂-integrin MoAb 60.3 (10 µg/mL). Values are displayed as % of PMA- or Mn²⁺-inducible adhesion and represent the mean ± SEM of three independent experiments. *P<0.001 as compared with control.

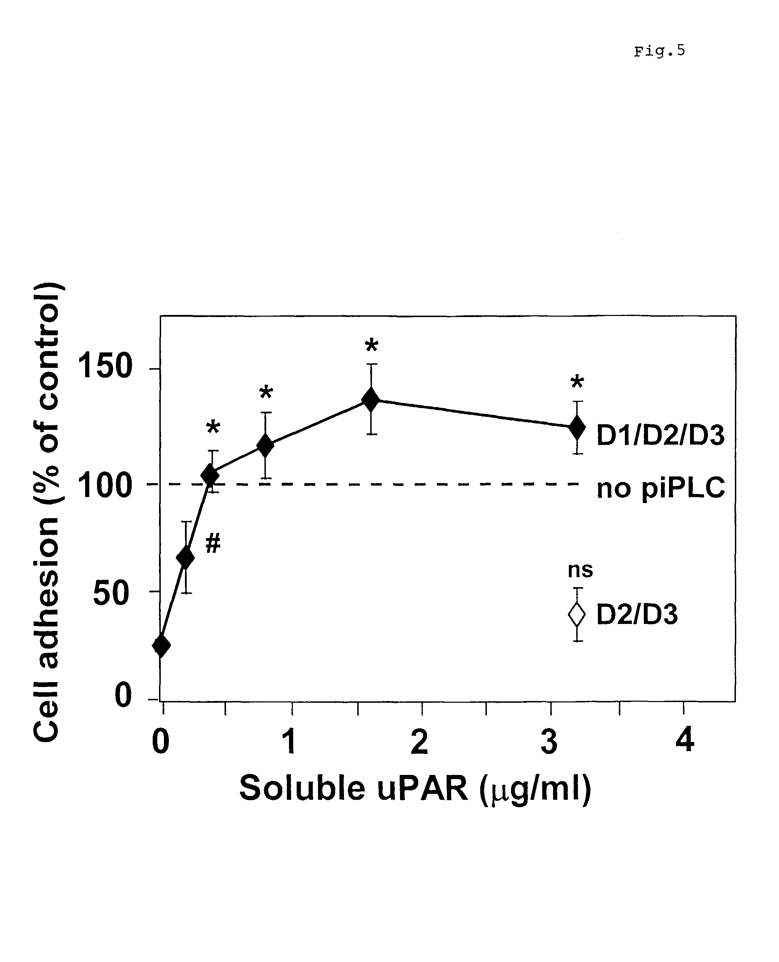

- Figure 5:: Intact soluble uPAR, but not its truncated form restores monocytic cell adhesion to endothelial cells. Adhesion of monocytic cells to HUVEC was performed as described in the legend to Fig. 4. pi-PLC-pretreated monocytic HL60 cells were incubated with increasing concentrations of soluble intact (D1/D2/D3) uPAR or with its truncated form (D2/D3) lacking domain 1 and subsequently stimulated by PMA. Values are displayed as % of PMA-inducible adhesion in non-pi-PLC-pretreated cells and one representative experiment (of three) is shown. ^{#}P<0.01 and *P<0.001 as compared with pi-PLC-pretreated cells without soluble uPAR.

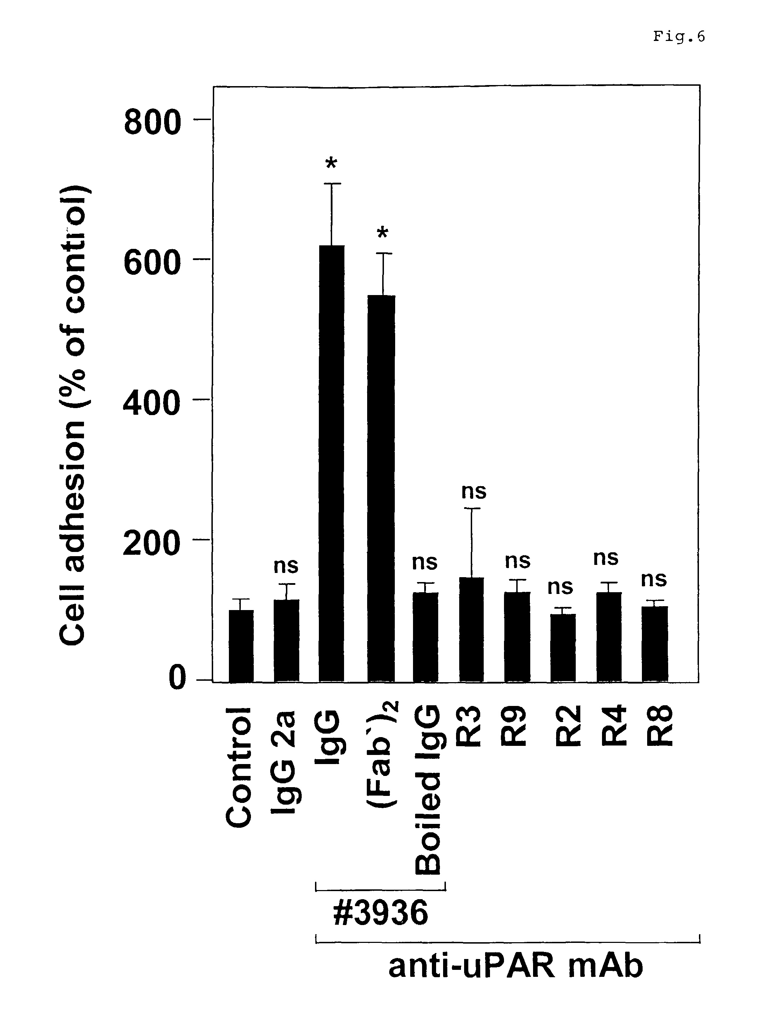

- Figure 6:: Effect of different anti-uPAR MoAbs on leukocytic cell adhesion to endothelial cells. Myelo-monocytic HL60 cells were incubated with different anti-uPAR MoAbs as indicated (20 µg/mL, each) (Fab')₂ fragment (12 µg/mL) or control IgG2a for 30 min; after washing, the adhesion assay was performed. Values are displayed as % of control (no antibody added) and represent the mean (± SEM) of three independent experiments. *P<0.001 as compared with control.

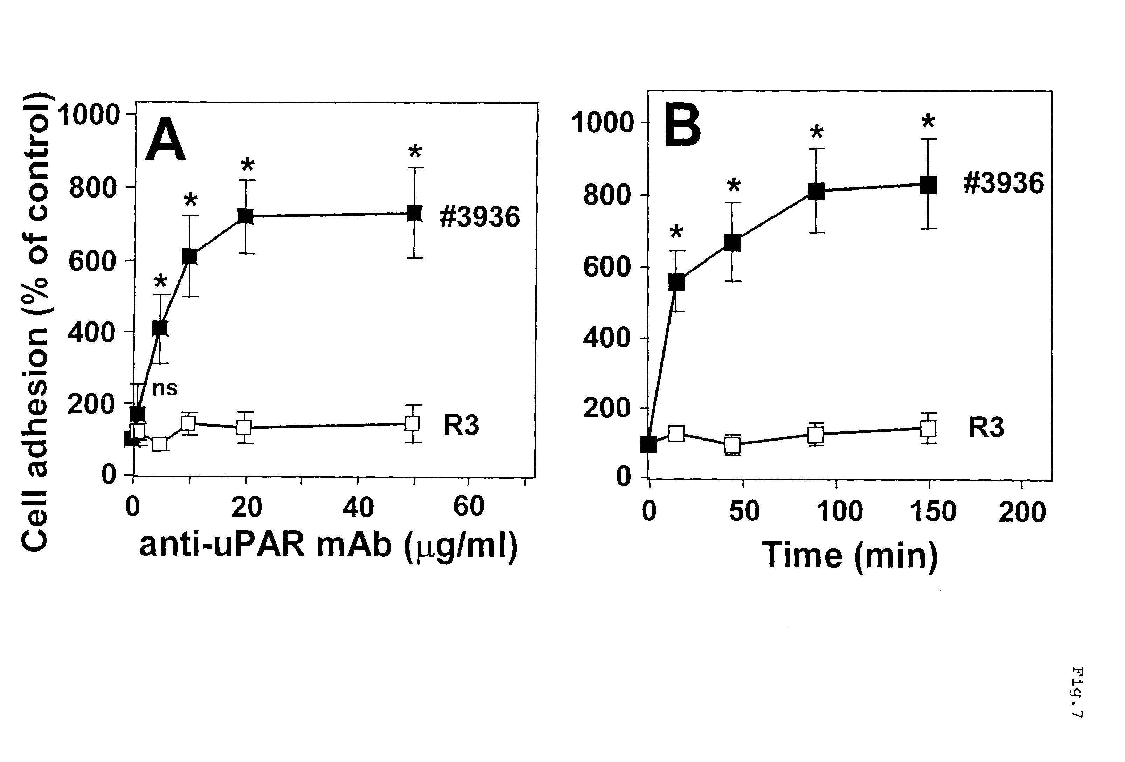

- Figure 7:: Anti-uPAR MoAb #3936 induces leukocyte cell adhesion to endothelial cells. MoAb #3936 (closed squares) or R3 (open squares) were added to myelo-monocytic HL60 cells in different concentrations as indicated for 1 h (A), or for different time intervals as indicated at a concentration of 10 µg/mL (B). Following washing, the adhesion assay was performed as described. Values (mean ± SEM, n=3) are displayed as % of control (no antibody added) and one representative experiment (of three) is shown., *P<0.001 as compared with control (no antibody added at time 0).

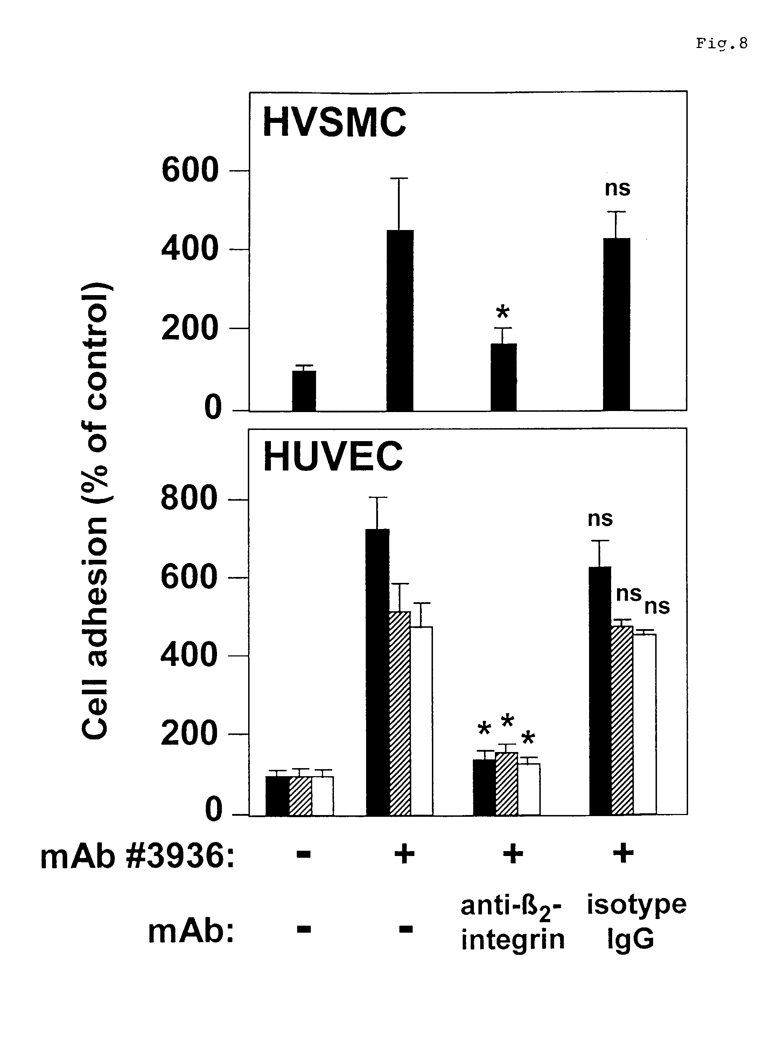

- Figure 8:: MoAb #3936 induces adhesion of leukocytes and neutrophils to endothelial and smooth muscle cells via β₂-integrins. Monocytic HL60 cells (black bars), monocytic U937 cells (hatched bars) and freshly isolated neutrophils (white bars) were pretreated with MoAb anti-uPAR #3936 (20 µg/mL) for 30 min as indicated. After washing, the adhesion assay was performed in the absence or presence of anti-β₂-integrin MoAb 60.3 or isotype control MoAb (10 µg/mL), as indicated. Values (mean ± SEM, n=3) are displayed as % of control (no antibody added) and express one (of three) representative experiment. *P<0.001 as compared with anti-uPAR-induced adhesion.

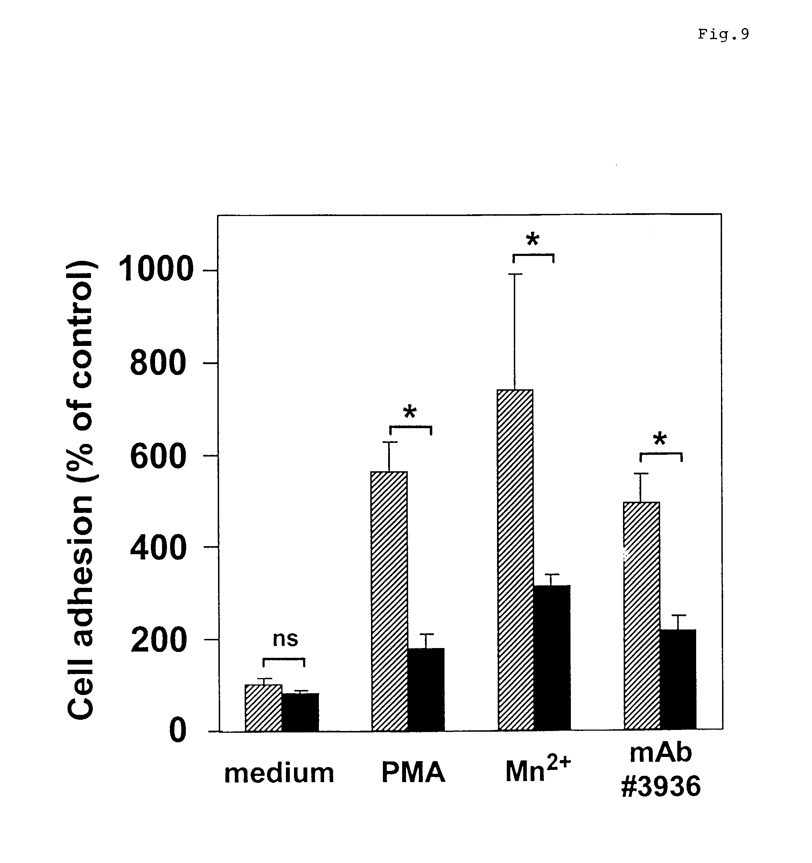

- Figure 9:: uPA interferes with leukocyte adhesion to endothelium. Myelo-monocytic HL60 cells were incubated in the presence (black bars) or absence (hatched bars) of inactivated uPA (50 nmol/L) for 30 min (37°C, 5% CO₂) before addition of medium, PMA (10 ng/mL), Mn²⁺(1.0 mmol/L) or MoAb anti-uPAR #3936 (10 µg/mL) for another 30 min, as indicated. After washing, the adhesion assay was performed. Values are displayed as % of untreated control (medium) and represent the mean (± SEM) of three independent experiments. *P<0.01.

### Examples

### 1. Materials and methods

### 1.1 Materials

Manganese chloride was obtained from Sigma (Munich, Germany), phorbol 12-myristate 13-acetate (PMA) from Gibco (Paisley, Scotland). Recombinant phosphatidylinositol-specific phospholipase C (pi-PLC) was from Oxford GlycoSystems (Abingdon, UK). Intact recombinant soluble uPAR as well as the chymotrypsin-cleaved truncated form lacking domain 1 were produced as described^{29,30}. uPA (Medac, Hamburg, Germany) inactivated by diisopropylfluorophosphate (Serva, Heidelberg, Germany) (DIP-uPA) was prepared as described³¹.

### 1.2 Antibodies

The following mouse anti-human uPAR monoclonal antibodies (MoAbs) were used: MoAb #3936 (IgG2a-type) (American Diagnostica, Greenwich, CT), which is known to block uPA binding; MoAbs R3 and R9 which recognize domain 1 and interfere with uPA binding; MoAbs R2, R4 and R8 which recognize domain 2 and 3 without influencing uPA-binding. The characteristics of MoAbs R2, R3, R4, R8 and R9 have been described previously³². Mouse anti-human β₂-chain (CD18) MoAb 60.3 (IgG2a-type) blocks β₂-integrin-mediated leukocyte adhesion to endothelium³³. Mouse anti-human IgG2a (Sigma) was used as isotype-matched control antibody. When necessary, FITC-conjugated mouse MoAbs anti-CD11 a (#25.3), anti-CD11b (Bear 1) and anti-CD18 (#7E4) (Immunotech, Hamburg, Germany) were used for flow cytometry.

The following reagents were used in animal experiments. Phycoerythrin (PE)-labeled streptavidin (Southern Biotechnology Associates, Inc., Birmingham, AL) and biotin-conjugated rat anti-mouse CD11 b (M1/70), Ly-6G (Gr-1) (RB6-8C5), and CD3e (145-2C11) (all from PharMingen, San Diego, CA). Anti-αLβ₂ integrin (CD11a) (FD441.8)³⁶ and YN1.1³⁷ (American Type Culture Collection, Rockville, MD) were affinity purified and dialyzed under endotoxin-free conditions.

(Fab')₂ fragments were generated using digestion by immobilized pepsin followed by protein A-Sepharose affinity chromatography (Pierce Chemical Co., Rockford, IL). Purity was controlled by polyacrylamide gel-electrophoretic analysis.

### 1.3 Cells

Human myeloid HL60 and U937 cell lines (German Collection of Microorganisms and Cell Cultures, Braunschweig, Germany) were cultured in RPMI-1640 supplemented with 10% (vol/vol) fetal calf serum, 1% sodium pyruvate, 1 mmol/L L-glutamine, 100 U/mL penicillin and 100 µg/mL streptomycin (all from Gibco). 24 hours prior to experiments monocytic differentiation was induced by addition of 50 ng/mL 1 α,25-dihydroxyvitamin D3 and 1 ng/mL transforming growth factor β1 (Biomol, Hamburg, Germany). Peripheral blood polymorphonuclear neutrophils were isolated by discontinuous density gradient centrifugation using Histopaque-1119 and -1077 (Sigma) as described by the manufacturer. An enrichment of at least 95% neutrophils was obtained as controlled by flow cytometry using forward and side scatter analysis and staining for CD15. Human umbilical vein endothelial cells (HUVEC) were isolated as described³⁴ and cultured (for 2-4 passages) in low serum endothelial cell growth medium (Promo Cell, Heidelberg, Germany) on gelatin-coated tissue-culture plastic. Human vascular smooth muscle cells (HVSMC) were isolated from aorta or saphenous vein by the explant method and characterized as described before³⁵. Early passage cells were cultured in smooth muscle cell medium (Promo Cell).

### 1.4 Adhesion Assays

HUVEC were seeded in gelatin-coated 48 well plates (Costar, Badhoevedorp, The Netherlands) 48 hours prior to the experiment. Confluency was confirmed by microscopic inspection before each experiment. HL60 or U937 cells were radiolabelled with 1 µCi/mL [methyl-³H]-thymidine (Amersham, Buckinghamshire, U.K.) for 24 hours and differentiated to monocytic cells (see above) for another 24 hours. These monocytic cells or freshly isolated neutrophils were washed twice in adhesion medium (serum-free RPMI 1640/Hepes 25 mmol/L), followed by different pretreatments (pi-PLC, soluble uPAR, uPA, different MoAbs, PMA, or Mn²⁺ ; for respective details see figure legends) and a washing step, and were added (7×10⁵/mL adhesion medium) to the prewashed HUVEC monolayers in the presence or absence of the blocking MoAb anti-β₂-integrin or isotype IgG (final concentration 10 µg/mL). After 30 min coincubation (37°C, 5% CO₂, 90% humidity), the plates were gently washed twice with adhesion buffer to remove non-adherent cells. Remaining adherent cells were lysed with 1 mol/L NaOH and quantitated in a β-counter. When flow cytometry was performed in parallel or neutrophil adhesion was tested, the number of adherent cells in 10 high power fields was counted using light microscopy. At least triplicate wells were run per test substance, and results are expressed as mean values ± SEM. The test principle for cell adhesion to HVSMC, which were seeded 7-9 days before the assay, was identical.

### 1.5 Peritonitis Assay

Peritonitis was induced in female UPAR^{-/-38} or wild-type mice by intraperitoneal injection of a solution of 4% (wt/vol) in Bacto Fluid Thioglycollate Medium (Difco Labs., Detroit, Ml). Endotoxin-free stock solutions were prepared at 100°C and subsequently heat-sterilized.

Mice obtained by shipping were generally kept (at the Basel Institute for Immunology) for at least 2 wk before the start of experiments to relieve stress. PBS alone or antibodies against ICAM-1, LFA-1 (200 µl of a 1 mg/mL solution each) were injected intravenously into the tail vein 30 min before induction of peritonitis. All reagents were endotoxin-free. After 4 or 24 h, respectively, mice were killed and peritoneal cells were suspended by injection of 5 ml PBS containing 2 mmol/mL EDTA and 50 µg/mL heparin into the peritoneum. The loaded mouse was shortly massaged and 4 ml of this lavage were collected and leukocytes were counted on a Coulter counter ZM equipped with a channelizer 256 (Coulter Corp., Miami, FL).

### 1.6 Flow cytometry

### Animal Model

The mouse leukocyte subpopulations were further analyzed by flow cytometry (Becton Dickinson, Heidelberg, Germany) using the biotin-coupled anti-mouse CD11b (integrin αM chain, Mac-1) anti-Gr-1 (anti-myeloid differentiation antigen Gr-1), or anti-CD3 (anti-CD3 TCR-associated complex). Fc receptors of leukocytes were blocked by preincubation of the cells for 30 min with 5% (vol/vol) rat and 5% (vol/vol) mouse serum in PBS. The secondary reagent PE-coupled streptavidin (dilution of 1:2,500) was incubated with the cells for 30 min.

### In vitro system

Cells (2.5x10⁵) were washed twice with Hepes buffered saline (HBS) and incubated with the indicated primary mouse-anti-human antibodies for 30 min on ice (for CD11b-detection at 21°C). Cells were washed again and resuspended in HBS containing fluorescein-conjugated F(ab')₂ fragment of goat anti-mouse IgG (Dianova, Hamburg, Germany). To test the effect of mouse antibodies on β₂-integrin expression FITC-conjugated monoclonal antibodies at a dilution of 1:50 were used. Mean fluorescence of 5000 cells was measured in a flow cytometer (Becton Dickinson, Heidelberg, Germany). Nonspecific fluorescence was determined using an isotype-matched mouse-lgG as primary antibody.

### 1.7 Statistical Analysis

Comparisons between group means were performed using multivalent analysis (ANOVA). Data represent mean ± SEM; P<0.05 was regarded as significant.

### 2. Results

### 2.1 Leukocyte Emigration in uPAR-deficient Mice.

Transendothelial migration of leukocytes to inflamed tissue depends on the interaction of the leukocyte with the vascular endothelium by β₂ integrins and ICAM-1. Thioglycollate-induced peritonitis is a reliable model to test leukocyte emigration into sites of acute inflammation. Disruption of the mouse ICAM-1-β₂ integrin interactions resulted in reduced leukocyte emigration in this model when compared with wild-type animals. Both uPAR-deficient and wild-type animals of the identical genotype (129 X C57/BL6 F1) were compared for leukocyte emigration in the peritonitis model. The number and types of leukocytes in the peripheral blood were identical in both sets of mice (data not shown). Lavages perfcrmed 4 (Fig.1) and 24 h (data not shown) after induction of peritonitis showed ∼50% reduction in counts of the total leukocyte population in uPAR-deficient mice when compared with wild-type animals (Fig.1). When animals were treated with anti-ICAM-1 or anti-LFA-1 antibodies at the time of induction of peritonitis, the number of emigrating leukocytes was further reduced by 50% in wild-type mice, but by only 30% in uPAR-deficient animals, suggesting that a major part of the initial lack of emigration was due to a perturbed β₂ integrin/lCAM-1 function. Analysis of the leukocyte subpopulations by flow cytometry using specific markers as indicated in Materials and Methods revealed that in uPAR-deficient mice granulocytes almost totally lost their ability to migate into the peritoneum after 4 and 24 h or inflammation (Fig.2). Myeloid lineage cells showed signficant reduction in recruitment after 4 h (∼ 55%) and 24 h (∼ 70%), whereas T lineage cells were hardly affected by the absence of uPAR after 4 h, but showed significant inhibition in emigration (∼ 60%) after 24 h (Fig.2). Consistently, administration of MoAbs demonstrated that lymphocyte recruitment after 4 h was largely independent of LFA-1-ICAM-1 interactions in contrast to recruitment after 24 h of inflammation.

To further specify those granulocytic subpopulations that were mostly affected, a differential cell staining (May-Grünwald-Giemsa) was performed (Fig.3). In uPAR-deficient mice, after 4 h, neutrophil and eosinophil recruitment was inhibited by >70% or 90%, respectively, and residual emigration was marginally affected by the administration of MoAbs against ICAM-1 or LFA-1, respectively. In contrast, in wild-type mice the recruitment of these two cell types was effectively blocked by these antibodies down to the level of emigrated cells in UPAR^{-/-} mice, suggesting that leukocyte recruitment in uPAR-deficient mice is diminished through impaired function of the β₂ integrin/ICAM-1 system. Basophil emigration into the inflamed peritoneum was not significantly affected in uPAR-deficient mice but was comparable to that in wild-type mice receiving anti-ICAM-1 or anti-LFA-1 MoAb, respectively. Comparable findings for granulocyte subpopulations were noted after 24 h of inflammation (data not shown). These data provide in vivo evidence for a functional consequence of the UPAR/β₂ integrin system in leukocyte adhesion/migration and present a new phenotype for uPAR-deficient mice.

### 2.2 Requirement of uPAR for Leukocyte Adhesion to Endothelium in Vitro

uPAR is expressed on circulating human blood cells, particularly on granulocytes, monocytes and activated T-cells. The human myeloid HL60 and U937 cell lines present many similarities to native mononuclear phagocytes, especially when differentiated by treatment with vitamin D3 and transforming growth factor-β1³¹. Consistently, this treatment induced a monocyte-specific surface antigen expression pattern for CD14, CD11b, CD18, and CD87 (uPAR) as measured by flow cytometry (data not shown). Adhesion of differentiated monocytic cells to endothelial cell monolayers was induced 6-8 fold by short term prestimulation with 10 ng/mL PMA or direct activation of β₂-integrins with 1.0 mmol/L Mn²⁺. The MoAb 60.3 directed against the β₂-chain of integrins blocked adhesion by 75-85% in both cases indicating that cell-to-cell contact was mainly β₂-integrin activation dependent (Fig. 4).

### 2.2.1 Soluble urokinase receptor rescues adhesion of pi-PLC-treated monocytic cells to HUVEC.

Pretreatment of leukocytes with phosphatidylinositol-specific phospholipase C (pi-PLC) removed ∼80% of uPAR from the cell surface as assessed by flow cytometry (not shown). This resulted in a 70-80% reduction of PMA- or a 65-70% reduction of Mn²⁺-induced leukocyte adhesion (Fig. 1). Readdition of intact soluble uPAR to the pi-PLC-pretreated cells for 10 min restored adhesion back to the level of non-pretreated cells. This UPAR-induced restoration of adhesion was abrogated by anti-CD18 indicating that the β₂-integrin-dependent adhesion was inducible by uPAR.

In particular, intact soluble uPAR increased monocytic cell adhesion to endothelial cells back to maximal levels in a dose-dependent manner, whereas addition of the truncated form of uPAR lacking the uPA binding domain D1 did not indicate a specific structural requirement for activation of β₂-integrin function (Fig. 5). In contrast to PMA that induces both increased surface expression as well as activation of β₂-integrins, flow cytometric analysis revealed no change in integrin expression after Mn²⁺-treatment (not shown) as described earlier⁹. Likewise, removal of uPAR did not affect surface expression of the αL-, αM- or β₂-chain of the integrin in resting or stimulated cells as analyzed by flow cytometry (not shown). Thus, the presence of uPAR appears to support adhesion by regulating integrin function rather than by quantitatively changing β₂-integrin levels on the cell surface.

### 2.2.2 Ligation of uPAR by a monoclonal antibody induces leukocyte adhesion to endothelial and smooth muscle cells via β₂-integrins

Since uPAR is directly involved in β₂-integrin-mediated leukocyte adhesion to the endothelium, we examined the consequences of uPAR occupancy on leukocyte adhesion to endothelial cells using the high affinity ligand uPA as well as different MoAbs against uPAR. Preincubation with an anti-uPAR MoAb (#3936) resulted in a dose- and time-dependent increase of adhesion reaching the same maximal level as achieved with PMA or Mn²⁺(Fig. 6). For undifferentiated HL60 or U937 cells, which show low surface expression of β₂ integrins and uPAR, respectively, the activating anti-uPAR MoAb increased adhesion only 1.5-2-fold (data not shown). Boiling of the antibody totally abrogated its adhesion-stimulating effect. In addition, (Fab')₂ fragments of MoAb #3936 had a very similar proadhesive effect as compared with the intact MoAb. Control isotype-matched MoAb IgG2a as well as other anti-uPAR mAbs directed against different epitopes of uPAR did not induce leukocyte adherence.

The kinetics of antibody ligation suggested that the uPAR/MoAb complex serves as a fast and effective trigger of integrin activation (Fig. 7). In contrast, the anti-uPAR MoAb R3 that has the same inhibitory effect on uPA binding to the receptor as MoAb #3936 was not able to promote leukocyte adhesion. The anti-uPAR MoAbs did not significantly change β₂-integrin expression (not shown) indicating that a functional conformational change of the adhesion receptor is responsible for the proadhesive effect.

Analogous to PMA- or Mn²⁺- induced β₂-integrin-dependent cell adhesion, MoAb 60.3 could abrogate anti-uPAR #3936-induced leukocyte adhesion to HUVEC indicating β₂-integrin dependency (Fig. 8). In addition, vitamin D3/TGFβ1 differentiated U937 cells and freshly isolated peripheral blood neutrophils responded to anti-uPAR MoAb #3936 in an identical manner indicating that this functional interaction of uPAR with β₂-integrins occurs in both monocytes and neutrophils.

### 2.3 Occupancy of uPAR by urokinase reduces β₂-integrin activation-dependent leukocyte adhesion to endothelium

The effect of uPA, the native ligand of uPAR, was tested on monocytic cell adhesion to endothelium (Fig. 9). While exogenous uPA had no significant effect on background adhesion, it significantly inhibited β₂-integrin-mediated adhesion in response to any of the three described stimulating pathways: Adhesion of leukocytes to endothelium achieved by cell activation via PMA, direct extracellular integrin activation via Mn²⁺or uPAR-ligation by the activating MoAb, respectively, was inhibited by active (not shown) as well as by enzymatically inactivate uPA. Thus, uPA binding to uPAR independently of its catalytic activity appears to control β₂-integrin-activation independent of the integrin activating stimulus. Since uPA or its inactivated isoform (DIP-uPA) did not alter the surface expression of the integrin αL-, αM-, or β₂-chains in resting or stimulated cells (not shown), we propose that the binding of uPA to uPAR prevents the induction of a conformational change leading to β₂-integrin activation.

### References

1. Springer, T.A. (1994), Traffic signals for lymphocyte recirculation and leukocyte emigration: The multistep paradigm, Cell 76: 301.
2. Carlos, T.M., Harlan, J.M. (1994), Leukocyte-endothelial adhesion molecules, Blood 84: 2068.
3. Larson, R.S., Springer, T.A. (1990), Structure and function of leukocyte integrins, Immunol. Rev. 114: 181.
4. Van der Vieren, M.H., Le Trong, C.L., Wood, P.F., Moore, L., St. John, D.E., Gallatin, W.M. (1995), A novel leukointegrin, αβ2 binds preferentially to ICAM-3, Immunity 3: 683.
5. Smith, C.W., Marlin, S.D., Rothlein, R., Toman, C., Anderson, D.C. (1989), Cooperative interactions of LFA-1 and Mac-1 with intercellular adhesion molecule-1 in facilitating adherence and transendothelial migration of human neutrophils in vitro, J. Clin. Invest. 83: 2008.
6. Lu, H., Smith, C.W., Perrard, J., Bullard, D., Tang, L.P., Shappell, S.B., Entman, M.L., Beaudet, A.L., Ballantyne, C.M. (1997), LFA-1 is sufficient in mediating neutrophil emigration in MAC-1-deficient mice, J. Clin. Invest. 99: 1340.
7. Couffinhal, T., Duplaa, C., Moreau, C., Lamaziere, J.-M.D., Bonnet, J. (1994), Regulation of vascular cell adhesion molecule-1 and intercellular adhesion molecule-1 in human vascular smooth muscle cells, Circ. Res. 74: 225.
8. Miller, L.J., Bainton, D.F., Borregaard, N., Springer, T.A. (1987), Stimulated mobilization of monocyte Mac-1 and p150,95 adhesion proteins from an intracellular vesicular compartment to the cell surface, J. Clin. Invest. 80: 535.
9. Altieri, D.C. (1991), Occupancy of CD11b/CD18 (Mac-1) divalent ion binding site(s) induces leukocyte adhesion, J. Immunol. 147:1891.
10. Skubitz, K.M., Campbell, K.D., lida, J., Skubitz, A.P.N. (1996), CD63 associates with tyrosine kinase activity and CD11/CD18, and transmits an activation signal in neutrophils, J. Immunol. 157:3617.
11. Zhou, M.-J., Todd, R.F., van der Winkel, J.G., Petty, H.R. (1993), Cocapping of the leukoadhesin molecules complement receptor type 3 and lymphocyte function-associated antigen-1 with Fcy receptor III on human neutrophils: Possible role of lectin-like interactions, J. Immunol. 150: 3030.
12. Bohuslav, J., Horejsi, V., Hansmann, C., Stöckl, J., Weidle, U.H., Majdic, O., Bartke, I., Knapp, W., Stockinger, H. (1995), Urokinase plasminogen activator receptor, β2-integrins, and src-kinases within a single receptor complex of human monocytes, J. Exp. Med. 181: 1381.
13. Xue, W., Kindzelskii, A.L., Todd, R.F., Petty, H.R. (1994), Physical association of complement receptor type 3 and urokinase-type plasminogen activator receptor in neutrophil membranes, J. Immunol. 152: 4630.
14. Ploug, M. Ronne, E., Behrendt, N., Jensen, A.L., Blasi, F., Dano, K. (1991), Cellular receptor for urokinase plasminogen activator. Carboxyl-terminal processing and membrane anchoring by glycosyl-phosphatidylinositol, J. Biol. Chem. 266: 1926.
15. Hoyer-Hansen, G., Ronne, E., Solberg, H., Behrendt, N., Ploug, M., Lund, L.R., Ellis, V., Dano, K. (1992), Urokinase plasminogen activator cleaves its cell surface receptor releasing the ligand-binding domain, J. Biol. Chem. 267: 18224.
16. Vassalli, J.D., Baccino, D., Belin, D. (1985), A cellular binding site for the Mr 55,000 form of the human plasminogen activator, urokinase, J. Cell. Biol. 100: 86.
17. Wei, Y., Waltz, D.A., Rao, N., Drummond, R.J., Rosenberg, S., Chapman, H.A. (1994), Identification of the urokinase receptor as an adhesion receptor for vitronectin, J. Biol. Chem. 269: 32380.
18. Kanse, S.M., Kost, C., Wilhelm, O.G., Andreasen, P.A., Preissner, K.T. (1996), The urokinase receptor is a major vitronectin binding protein on endothelial cells, Exp. Cell. Res. 224: 344.
19. Ossowski, L., Clunie, G., Masucci, M., Blasi, F. (1993), In vivo paracrine interaction between urokinase and its receptor: effect on tumor cell invasion, J. Cell. Biol. 115: 1107.
20. Cao, D., Mizukami, I.F., Garni Wagner, B.A., Kindzelskii, A.L., Todd, R.F., Boxer, L.A., Petty, H.R. (1995), Human urokinase-type plasminogen activator primes neutrophils for superoxide anion release. Possible roles of complement receptor type 3 and calcium, J. Immunol. 154: 1817.
21. Dumler, I., Petri, T., Schleuning, W.-D. (1993), Interaction of urokinase-type plasminogen activator (u-PA) with its cellular receptor (u-PAR) induces phosphorylation on tyrosine of a 38 kDA protein, FEBS Lett. 322: 37.
22. Busso, N., Masur, S.K., Lazega, D., Waxman, S., Ossowski, L. (1994), Induction of cell migration by pro-urokinase binding to its receptor: Possible mechanism for signal transduction in human epithelial cells, J. Cell. Biol. 126: 259.
23. Resnati, M., Guttinger, M., Valcamonica, S., Sidenius, N., Blasi, F., Fazioli, F. (1996), Proteolytic cleavage of the urokinase receptor substitutes for the agonist-induced chemotactic effect, EMBO J. 1 5: 1572.
24. Rao, N.K., Shi, G.-P., Chapman, H.A. (1995), Urokinase receptor is a multifunctional protein: influence of receptor occupancy on macrophage gene expression, J. Clin. Invest. 96: 465.
25. Petty, H.R., Todd, R.F. III (1996), Integrins as promiscuous signal transduction devices, Trends Immunol. Today 5: 209.
26. Wei, Y., Lukashev, M., Simon, D.I., Bodary, S.C., Rosenberg, S., Doyle, M.V., Chapman, H.A. (1996), Regulation of integrin function by the urokinase receptor, Science 273: 1551.
27. Sitrin, R.G., Todd, R.F., Petty, H.R., Brock, T.G., Shollenberger, S.B., Albrecht, E., Gyetko, M.R. (1996), The urokinase receptor (CD87) facilitates CD11B/CD18-mediated adhesion of human monocytes, J. Clin. Invest. 97: 1942.
28. Simon, D.I., Rao, N.K., Xu, H., Wei, Y., Majdic, O., Ronne, E., Kobzik, L., Chapman, H.A. (1996), Mac-1 (CD11b/CD18) and urokinase receptor (CD87) form a functional unit on monocytic cells, Blood 88: 3185.
29. Behrendt, N., Ronne, E., Ploug, M., Petri, T., Lober, D., Nielsen, L.S., Schleuning, W.-D., Blasi, F., Appella, E., Dano, K. (1990), The human receptor for urokinase plasminogen activator. NH2-terminal amino acid sequence and glycosylation variants, J. Biol. Chem. 265: 6453.
30. Behrendt, N., Ploug, M., Patthy, L., Houen, G., Blasi, F., Dano, K. (1991), The ligand-binding domain of the cell surface receptor for urokinase-type plasminogen activator, J. Biol. Chem. 266: 7842.
31. Waltz, D.A., Sailor, L.Z., Chapman, H.A. (1993), Cytokines induce urokinase-dependent adhesion of human myeloid cells. A regulatory role for plasminogen activator inhibitors, J. Clin. Invest. 91: 1541.
32. Ronne, E., Behrendt, N., Ellis, V., Ploug, M., Dano, K., Hoyer-Hansen, G. (1991), Cell-induced potentiation of the plasminogen activation system is abolished by a monoclonal antibody that recognizes the NH2-terminal domain of the urokinase receptor, FEBS Lett. 288: 1.
33. Wallis, W.J., Hickstein, D.D., Schwartz, B.R., June, C.H., Ochs, H.D., Beatty, P.G., Klebanoff, S.J., Harlan, J.M. (1986), Monoclonal antibody-defined functional epitopes on the adhesion-promoting glycoprotein complex (CDw18) of human neutrophils, Blood 67: 1007.
34. Jaffe, E.A., Nachman, R.L., Becker, C.G., Minick, C.R. (1973), Culture of human endothelial cells derived from umbilical veins, J. Clin. Invest. 52: 2745.
35. Kanse, S.M., Wijelath, E., Kanthou, C., Newman, P., Kakkar, V.V. (1995), The proliferative responsiveness of human vascular smooth muscle cells to endothelin correlates with endothelin receptor density, Lab. Invest. 72: 376.
36. Sanchez-Madrid, F., P. Simon, S.Thompson and T.A.Springer, 1983. Mapping of antigenic and functional epitopes on the α and β subunits of two related mouse glycoproteins involved in cell interactions, LFA-1 and Mac-1. J.Exp.Med. 158:586-602.
37. Prieto, J., F.Takei, R.Gendelman, B.christenson, P.Biberfled, and M.Patarroyo, 1989. MALA-2, mouse homologue of human adhesion molecule ICAM-1 (CD54). Eur.J.Immunol.19:1551-1557.
38. Bugge, T.H., T.T.Suh, M.J.Flick, C.C.Daugherty, J.Romer, H.Solberg, V.Ellis, K.Dano, and J.L.Degen. 1995. The receptor for urokinase-type plasminogen activator is not essential for mouse development or fertility. J.Biol.Chem.270:16886-16894.
39. Preissner, K.T., May, A.E., Wohn, K.D., Gerner, M., Kanse, S.M. (1997), Molecular Crosstalk Between Adhesion Receptors and Proteolytic Cascades in Vascular Remodelling, Thromb. Haemostas.78:88.

## Claims

1. Use of urokinase receptor (uPAR) and ligands thereof for the manufacture of a medicament to modulate the β₂-integrin-mediated cell-to-cell adhesion.

2. Use of claim 1 to modulate the adhesion of leukocytes.

3. Use of claim 2 to modulate the adhesion of cells selected from lymphocyte-derived cells, granulocytes, e.g.neutrophils or eosinophils and monocytes.

4. Use of claim 1 to modulate the adhesion of tumor cells.

5. Use of claim 4 to modulate the adhesion of metastating tumor cells.

6. Use of any of claims 1 to 5 to modulate the adhesion of single cells in the blood stream to endothelial cells and muscle cells.

7. Use of claim 6 to modulate the adhesion to cells selected from the group consisting of vascular endothelial cells and smooth muscle cells.

8. Use of any of claims 1 to 7 to stimulate the cell adhesion.

9. Use of any of claims 1 to 7 to inhibit the cell adhesion.

10. Use of any of claims 1 to 9, wherein the medicament comprises as an active agent a urokinase receptor ligand.

11. Use of claim 10, wherein the urokinase receptor ligands are selected from the group consisting of anti-uPAR antibodies, urokinase and uPAR-binding fragments thereof and synthetic peptide derivatives or low molecular compounds capable of binding uPAR.

12. Use of any of claims 1 to 11 for the treatment of inflammatory conditions in immune diseases, or vascular diseases.

13. Use of claim 12 for the treatment of peritonitis, hyperinflammatory conditions or atherogenesis.

14. Use of any of claims 1 to 11 for the treatment of invasive processes, e.g. tumors, particularly for the inhibition of tumor metastasis.

15. A method for modulating β₂-integrin-mediated cell-to-cell adhesion in a subject, comprising administering a urokinase receptor or a ligand thereof in a therapeutically effective dose.
